# EUROPEAN PATENT SPECIFICATION

(11) **EP 3 500 246 B1**
(45) Date of publication and mention of the grant of the patent: **04.08.2021**
(21) Application number: 16775356.5
(22) Date of filing: 18.08.2016
(51) Int. Cl.: A61K 9/20, A61K 9/28, A61K 31/198, A61K 31/277

(54) **ANTIPARKINSON TABLET FORMULATION WITH IMPROVED DISSOLUTION PROFILE**
TABLETTENFORMULIERUNG GEGEN PARKINSON MIT VERBESSERTEM AUFLÖSUNGSPROFIL
FORMULATION DE COMPRIMÉ ANTIPARKINSONIEN À PROFIL DE DISSOLUTION AMÉLIORÉ

(43) Date of publication of application: 26.06.2019
(73) Proprietor: Ilko Ilaç Sanayi Ve Ticaret Anonim Sirketi, 34885 Sancaktepe/Istanbul (TR)
(72) Inventor: ÖNCEL, Hatice, 34885 Istanbul (TR); ÇAPAN, Yilmaz, Ankara (TR); PINARBASLI, Onur, Ankara (TR); AKANSEL, Sibel, Ankara (TR); SARRAÇOGLU, Nagehan, Ankara (TR)
(74) Representative: Bulut, Pinar
(86) International application number: PCT/TR2016/050292
(87) International publication number: WO 2018/034626

(56) References cited:
- EP-A1- 2 308 487
- WO-A1-2009/098661

## Description

### Technical field:

The present invention relates to develop pharmaceutical fixed dose combination of levodopa, carbidopa and entacapone or salts thereof,wherein by using low particle size distributionentacapone shows very rapidly dissolution profile. This invention also relates to atime and cost effective process of immediate release tablet comprising levodopa, carbidopa and entacapone with using common granule.The invention relates to a method of producing said tablet. The composition of the invention exhibits bioequivalence to commercially available levodopa, carbidopa and entacapone combination formulation marketed under the trade name Stalevo200®.

### Prior Art:

Levodopa, an aromatic amino acid, is a white crystalline compound. Slightly soluble in water, but solubility increases below pH 3 and above pH 8. It is classified as Class I, high solubility and high permeability, according to Biopharmaceutics Classification System (BCS). It is designated chemically as (+L-α-amino-β-(3,4-dihydroxybenzene)propanoic acid. Its empirical formula is C₉H₁₁NO₄ and its structural formula is:

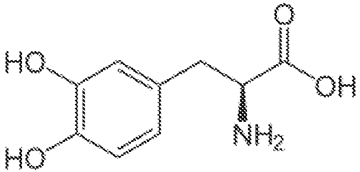

Carbidopa, an inhibitor of aromatic amino acid decarboxylation, is a white, crystalline compound. Slightly soluble in water, but dissolve readily in dilute mineral acids, solubility increases above pH 7. According to BCS the solubility of carbidopa is considered high. It is designated chemically as (-)-L- (α-hydrazino-(α-methyl-β-(3,4-dihydroxybenzene) propanoic acid. Its empirical formula is C₁₀H₁₄N₂O₄ and its structural formula is: 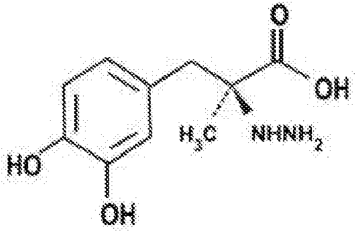 Entacapone, an inhibitor of catechol-O -methyltransferase (COMT) and yellow crystalline powder. It is used in the treatment of Parkinson's disease as an adjunct to levodopa/ carbidopa therapy. It is practically insoluble in water, however, solubility is slightly higher above pH 7. It is Class IV (Low Solubility - Low Permeability Drugs) under the BCS and poses problems of low solubility, low dissolution rate and hence bioavailability. The chemical name of entacapone is (E)-2-cyano-3-(3,4-dihydroxy-5-nitrophenyl)-N,N-diethyl-2-propenamide. Its empirical formula is C₁₄ H₁₅ N₃ O₅ and its structural formula is:

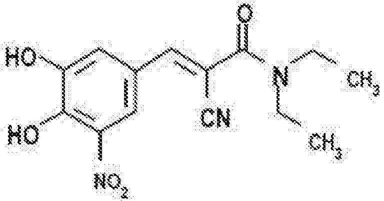

US 20080051459 discloses a method of treating Parkinson's disease comprising administering pharmaceutically effective amount of a composition comprising levodopa.

Entacapone is described in U.S. Pat. No. 5,446,194 as a catechol-O-methyltransferase (COMT) inhibitor. Enteral and parenteral routes of administration of entacapone are discussed in U.S. Pat. No. 5,446,194.

US 4,963,590 provides a pharmaceutical composition comprising entacapone and pharmaceutically acceptable carrier.

US 2007/0275060 disclose an extended release tablet comprising an extended release composition comprising levodopa; and an immediate or rapid release composition comprising carbidopa.

U.S. Patent No. 6,500,867 and 6,797,732 disclose oral solid tablet compositions comprising entacapone, levodopa and carbidopa, or pharmaceutically acceptable salts or hydrates thereof, and a pharmaceutically acceptable excipient.

US 7,094,427 and US 20040166159 disclose a composition comprising immediate release and extended release component.

WO07/073702 discloses a multi-layered tablet providing three different release profiles.

As used herein, 'fixed-dose combination (FDC)' refers to a combination of two or more active pharmaceutical ingredients (APIs) combined in a single dosage form, which is manufactured and distributed in fixed doses such as a tablet or oral dosage form.

Parkinsonism medication needs to be taken several times a day to keep the patients without symptoms. Therefore, patient compliance can be improved significantly by using a fixed dose combination of entacapone, levodopa, and carbidopa instead of taking two separate tablets, i.e., an entacapone tablet and a levodopa-carbidopa tablet, several times a day. This is especially important for parkinsonism patients with tremor and old age.

The triple combination of levodopa, carbidopa and entacapone is available as immediate release composition in six different doses. For example Stalevo 50 (containing 12.5 mg of carbidopa, 50 mg of levodopa and 200 mg of entacapone), Stalevo 75 (containing 18.75 mg of carbidopa, 75 mg of levodopa and 200 mg of entacapone), Stalevo 100 (containing 25 mg of carbidopa, 100 mg of levodopa and 200 mg of entacapone), Stalevo 125 (31.2575 mg of carbidopa, 125 mg of levodopa and 200 mg of entacapone), Stalevo 150 (containing 37.5 mg of carbidopa, 150 mg of levodopa and 200 mg of entacapone) and Stalevo 200 (containing 50 mg of carbidopa, 200 mg of levodopa and 200 mg of entacapone).

EP 2252284 B1 discloses a single oral dose pharmaceutical composition comprising a combination of entacapone, levodopa and carbidopa or salts thereof along with one or more sugar alcohols; wherein the entacapone is co-micronized with one or more sugar alcohols.

US 2009/0155369 A1 discloses a solid pharmaceutical composition for oral intake comprising entacapone, levodopa and carbidopa or pharmaceutically acceptable salts or hydrates thereof characterized in that entacapone or pharmaceutically acceptable salt thereof is in the form of granules and in that levodopa and carbidopa do not form part of entacapone's granules. Inventors found that this formulation should be bilayer tablet for adequate stability.

US6500867 B1 has mentioned an oral solid tablet composition comprising pharmacologically effective amounts of entacapone, levodopa and carbidopa, or pharmaceutically acceptable salts or hydrates thereof, wherein a substantial portion of the carbidopa or pharmaceutically acceptable salt or hydrate thereof is separated from the entacapone and levodopa or pharmaceutically acceptable salts or hydrates thereof in the tablet, and comprising at least one pharmaceutically acceptable excipient being a sugar alcohol or starch, or a sugar alcohol and starch. A way has been found to increase the bioavailability of carbidopa from an oral solid composition comprising entacapone, levodopa, and carbidopa is to add carbidopa separately, for instance by granulating first levodopa and entacapone together and then adding carbidopa to these granules separately.

This combination is marketed under six different doses as mentioned above. Producing six different doses with the manufacturing method described in US 6500867 B1 gives some difficulties while preparing each granule for each dose formulation.

As solubility problem of entacapone, it is classed in Class IV under the Biopharmaceutics Classification System (BCS) and poses problems of low solubility, low dissolution rate and hence bioavailability.

Therefore, it is still a need for developing pharmaceutical compositions which incorporate levodopa, carbidopa and entacapone to suggest a new solution to solve the solubility problem of entacapone while having adequate stability and create a process that can be applied at an industrial level simply and cost-effectively.

### Description of the Invention:

The aim of present invention is to provide pharmaceutical compositions comprising levodopa, carbidopa and entacapone or salts thereof for oral administration to solve the solubility problem of entacapone with using low particle size raw material while having adequate physical stability. In addition, it is an aim of the present invention to provide a time and cost-effective process by using common granule for preparing said pharmaceutical compositions, particularly tablets, by means of a process that can be applied at an industrial level with low energy costs and which does not subject the active ingredient to aggressive formulation conditions which can entail the loss of stability of the product. A manufacturing process does not form part of the claims.

The main feature of the present invention is to provide a pharmaceutical tablet composition comprising levodopa, carbidopa and entacapone or pharmaceutically acceptable salts or hydrates thereof and pharmaceutically acceptable excipient characterized in that; carbidopa and levodopa are granulated together and entacapone is granulated separately with one or more pharmaceutically acceptable excipients by wet granulation method, wherein median particle size distribution d(50) of entacapone or pharmaceutically acceptable salts or hydrates is about 2 µm when measured by the method of laser diffraction in a dry dispersion system.

Entacapone used as active pharmaceutical ingredient is practically insoluble in water. The term 'practically water insoluble' refers having a solubility of about more than 10.000 parts of solvent required to solubilize one part of drug. Because of this property, dissolution of the product & bioavailability is always a challenge to develop the product in formulation. Due to the poor solubility, the dissolution rate of entacapone can be a limiting factor for the absorption of entacapone in the gastro-intestinal tract. In order to facilitate the absorption to the patient, entacapone having a certain reduced particle size is preferably used in the dosage forms of the invention. Entacapone particles of reduced particle size, however, have tended to cause manufacturing problems such as poor flowability and agglomeration which may result in compromised content uniformity.

The particle size has a profound influence on almost every step in tablet manufacturing, including mixing, granulation, compression, and coating. Besides the manufacturability of tablets, the dissolution rate, which is proportional to surface area of drugs, is largely dependent on particle size distribution of drug particles.

Particle size distribution test is applied by the method of laser diffraction in a dry dispersion system (Malvern Mastersizer-2000, single narrow mode). Laser diffraction measures particle size distributions by measuring the angular variation in intensity of light scattered as a laser beam passes through a dispersed particulate sample. Large particles scatter light at small angles relative to the laser beam and small particles scatter light at large angles. The angular scattering intensity data is then analyzed to calculate the size of the particles responsible for creating the scattering pattern, using the Mie theory of light scattering. The particle size is reported as a volume equivalent sphere diameter.

A common approach to define the distribution width is to cite three values on the x-axis as d10, d50 and d90. The d50, named as median, has been defined as the diameter where 50 percent of the distribution lies below this value. Similarly, d90 has been defined as 90 percent of the distribution lies below, and 10 percent of the population lies below the d10.

Potential problems related to insolubility of entacapone active substance can be solved by inventors by using specific particle size of entacapone as well as a proper method for the preparation of the oral dosage form.

In this present invention, median particle size distribution (d50) of entacapone is less than 5 µm, preferably about 2 µm, thus solubility and bioavailability problems of entacapone are being solved, but this causes deterioration of the flow properties of entacapone. When all doses are considered, the percentage of entacapone is more in the low strengths and hence it is necessary to improve the flow of entacapone. Therefore, entacapone is granulated with pharmaceutically suitable excipients by wet granulation. Wet granulation also improves the solubility of entacapone.

Levodopa, carbidopa and entacapone combination has six different doses in the market. When levodopa and entacapone are granulated together with suitable excipients and carbidopa granules are added separately to these granules, granulation should be done separately for each six different doses. This situation is time consuming for industrial production.

In order to realize the aim of the present invention which is developing a time and cost effective manufacturing method with comprising the technical quality of the product, a common granule should be used instead of preparing granules separately for each doses. Entacapone dose is fixed in this formulation as shown in table below and also levodopa and carbidopa have a fixed 4 to 1 ratio, provided dose varies. By using this situation, inventors designed a new process model with using common granule.

| **Levodopa (mg)** | **Carbidopa (mg)** | **Entacapone (mg)** |
|---|---|---|
| 200 | 50 | 200 |
| 150 | 37.5 | 200 |
| 125 | 31.25 | 200 |
| 100 | 25 | 200 |
| 75 | 18.75 | 200 |
| 50 | 12.5 | 200 |
| 200 | 50 | 200 |
| 150 | 37.5 | 200 |
| 125 | 31.25 | 200 |
| 100 | 25 | 200 |
| 75 | 18.75 | 200 |
| 50 | 12.5 | 200 |

In the present invention levodopa and carbidopa are granulated together with suitable excipients by wet granulation technique and entacapone is granulated separately. These two granules are common granules for six doses of pharmaceutical product. Required amount of dose proportional levodopa - carbidopa granule for each dose are mixed with entacapone granule which has a fixed dose. This manufacturing method facilitates the production of the entire dose and process validations. Therefore six different doses are produced effectively with preventing loss of time and production costs compared to production methods having granulation of levodopa and carbidopa together.

Through this method, which has common unit formula and wet granulation process for all doses, manufacturing costs and time are reduced. Without the need for separate process validation for each dose, common granule process validation is sufficient.

In a further embodiment, levodopa, carbidopa and entacapone are granulated with pharmaceutically acceptable diluents, fillers, disintegrants and binders.

In the preferred embodiments of this invention, the solid dosage form is a tablet preferably film coated tablet. The amount of levodopa, carbidopa and entacapone are 50 to 200 mg, 12.5 mg to 50 mg and 200 mg respectively.

The tablet composition according to present invention comprises 30 to 75 wt.%, preferably 50 to 60 wt.%, of active ingredients as levodopa, carbidopa and entacapone or pharmaceutically acceptable salts or hydrates; 1 to 10 wt.%, preferably 3 to 5 wt.%., of wet granulation binder; 15 to 40 wt.%, of diluents and 0 to 30 wt.%, preferably 5 to 15 wt.%, of disintegrant.

Examples of suitable binders include starch, gums, pregelatinized starch, polyvinyl pyrrolidone (PVP), copovidone, cellulose derivatives, such as hydroxypropylmethylcellulose, hydroxypropylcellulose, and carboxymethylcellulose and their salts.

Examples of suitable diluents include lactose, lactose monohydrate, mannitol, sucrose, maltodextrin, dextrin, maltitol, sorbitol, xylitol, powdered cellulose, cellulose gum, microcrystalline cellulose, starch, calcium phosphate, or metal carbonate.

Examples of suitable lubricants include talc, magnesium stearate, calcium stearate, polyethylene glycol, hydrogenated vegetable oils, stearic acid, sodium stearyl fumarate.

Examples of suitable disintegrants include starch, croscarmellose sodium, crosspovidone, sodium starch glycolate and polacrilin potassium.

### Example 1

Levodopa-Carbidopa-Entacapone Film Coated Tablets formulations are representatives of the preferred compositions of the present invention. Example 1 is prepared according to the unit formula below:

**Table 1. Unit formula of Levodopa-Carbidopa-Entacapone Film Coated Tablets**

| **Igredients** | **%** |
|---|---|
| **Core tablet** | |
| Levodopa | 25.00 |
| Entacapone | 25.00 |
| Carbidopa monohydrate | 6.80 |
| Povidone | 5.00 |
| Starch | 12.70 |
| Mannitol | 9.00 |
| Crosscarmellose sodium | 15.00 |
| Magnesium stearate | 1.50 |
| Total | 100.00 |
| Film coating | 2.00 |

### Dissolution Profiles:

Dissolution testing measures the portion (%) of levodopa, carbidopa and entacapone that has been released from tablets and has dissolved in the dissolution medium of specified dissolution conditions during 60 minutes. The dissolution profiles of the products of the invention containing levodopa, carbidopa and entacapone are compared to the dissolution profiles of the references products containing levodopa, carbidopa and entacapone in specified dissolution conditions.
Dissolution medium for entacapone(FDA): pH 5.5, Apparatus I (Basket), phosphate buffer, 900 mL, 125 rpm
Dissolution medium for levodopa and carbidopa (FDA): pH 1.2, Apparatus I (Basket), 0.1 N HCI, 750 mL, 50 rpm

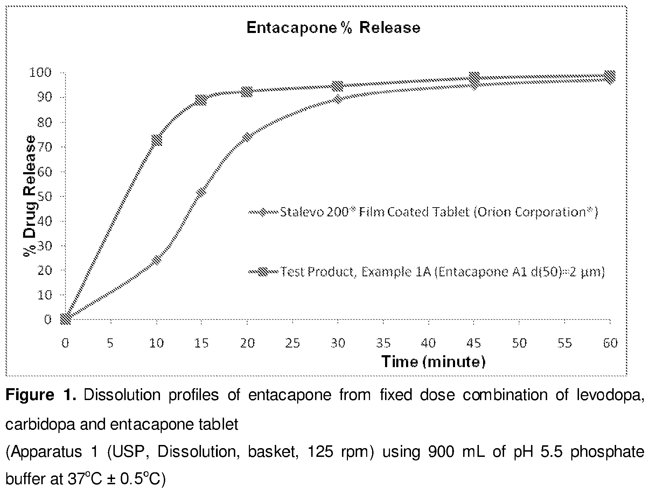

The invention is further clarified by testing two different particle size of entacapone, which are intended to be purely exemplary of the invention.Tablets prepared with entacapone A2 having median particle size distribution d(50) about 15 µm show similar dissolution profile with entacapone profile of Stalevo® 200 Film Coated Tablet (Orion Corporation®). Whereas tablets prepared by Example 1 with entacapone A2 having median particle size distribution d(50) about 2 µm give very rapidly dissolving profile of entacapone which is not expected result due to its solubility problems. The composition exhibits a dissolution profile such that at least 85% of the entacapone is released within 15 minuteswherein the release rate is measured in Apparatus I (USP, Dissolution, basket, 125 rpm) using 900 mL of pH 5.5 phosphate buffer at 37°C + 0.5°C. Dissolution datacan be seen at Table 1 and dissolution profile is at Figure 1. Very rapidly dissolving of an active substance can be defined as ≥ 85% dissolved in ≤ 15 minutes.

Using entacapone A1 having median particle size distribution (d50) about 2 µm gave a surprising effect on dissolving property of entacapone. Potential problems related to insolubility of entacapone active substance can be solved by using entacapone having median particle size distribution (d50) not more 5 µm, especially about2 µm.

**Table 2:Comparative dissolution data of Stalevo 200® vs Test product prepared by Example 1 with entacapone A1 vs Test product prepared by Example 1 with entacapone A2 (Dissolution in Apparatus I (USP, Dissolution, basket, 125 rpm) using 900 mL of pH 5.5 phosphate buffer at 37°C + 0.5°C)**

| **Product** | **% Release** | | | | |
|---|---|---|---|---|---|
| | **10 min** | **15 min** | **30 min** | **45 min** | **60 min** |
| Stalevo 200® (Orion Corporation) | 24.24 | 51.82 | 89.17 | 94.90 | 97.20 |
| Test Product, Entacapone A1 (d(50)=2 µm) | 72.54 | 88.90 | 94.62 | 97.88 | 98.91 |
| Test Product, Entacapone A2 (d(50)=15 µm) | 18.60 | 45.31 | 89.93 | 96.33 | 98.67 |

Although it is known that reduction in particle size leads to solubility and bioavailability increase, these parameters are not always adequate. For example, the bioavailability of micronized progesterone is not adequate and should be improved, for example with a dispersion in carnauba wax. Such a technique is described in International Publication No. (PCT) WO 8902742. Thus, it appears that the properties of a substance treated by micronization or grinding, in particular its solubility and its bioavailability, are not predictable and contradictory results may be obtained. In the present invention, although 15 µm and 2 µm are not a great difference in particle size, a sustantial difference was observed between these two particle size in terms of their solubility and dissolution profiles.

In the mechanism of drug, levodopa is the immediate precursor to dopamine. Entacapone is a selective, reversible catechol-O-methyltransferase (COMT) inhibitor that increases the bioavailability of levodopa. Valkovic P. (2005) indicates that the catechol-O-methyltransferase (COMT) inhibitors can prevent levodopa-induced elevation of homocysteine concentrations by reducing the O-methylation of levodopa. Keränen, T. (1993) states that in vivo biochemical effects of entacapone indicate that it is an orally active COMT inhibitor and that it may improve the therapeutic efficacy of levodopa in Parkinson's disease.As can be understood that entacapone improves the symptomatic efficacy of levodopa. In accordance with the present invention,tablets prepared by Example 1 procedure with entacapone A1 having particle size distribution d(50) about 2 µm getsvery rapid dissolution profile such that at least 85% of the entacapone is released within 15 minutes. This situation may improve the efficacy of levodopa much better because of higher concentration of entacapone atinitial times. This high concentration of entacapone may reduce O-methylation of levodopa more effectively. The very rapidly dissolution property of entacapone may also extend and enhance the therapeutic efficacy of levodopa in Parkinson's disease. Also bioequivalence results readily show that the present invention with very rapidly dissolving entacapone reach tmax before Stalevo 200®, which is another technical result of a very rapid dissolving property (tmax for Stalevo 200®: 1.69±1.13, tmax for test product: 1.38±1 .12).

Bioequivalence studies were carried out between Stalevo 200® and the composition of the present invention. The study was monitored in terms of Cmax, AUC₀₋ₜ, AUC_{0-∞}, and time to reach maximum plasma concentration (tmax). The present invention is bioequivalent to commercially available combination of levodopa, carbidopa, entacapone reference product (Stalevo 200®).

US 2009/0155369 A1 and US6500867 B1 patents have mentioned about stability problem of levodopa, carbidopa and entacapone tablets. In a further aspect of the invention, the unit dosage form of formulation according to the invention is physically and chemically stable. Stability of the tablets can be measured at accelerated as well as at long term storage conditions for periods of several weeks. Experiments can be performed at different temperatures and humidities. The oral pharmaceutical compositions of the present invention which are prepared according to Example 1 were subjected to accelerated stability studies at 40 ±2°C, 75±5 % RH conditions. Inventors have found that this product is stable under accelerated condition which is 40±2°C, 75±5 % RH for 6 months.

## Claims

1. A pharmaceutical tablet composition comprising levodopa, carbidopa and entacapone or pharmaceutically acceptable salts or hydrates thereof and pharmaceutically acceptable excipient **characterized in that**; carbidopa and levodopa are granulated together and entacapone is granulated separately with one or more pharmaceutically acceptable excipients by wet granulation method, wherein median particle size distribution d(50) of entacapone or pharmaceutically acceptable salts or hydrates is about 2 µm volume equivalent when measured by the method of laser diffraction in a dry dispersion system.

2. The pharmaceutical tablet composition according to claim 1, wherein the composition exhibits a dissolution profile such that at least 85% of entacapone is released within 15 minutes; wherein the release rate is measured in Apparatus 1 (USP, Dissolution, basket, 125 rpm) using 900 mL of pH 5.5 phosphate buffer at 37°C ± 0.5°C.

3. The pharmaceutical tablet composition prepared according to claim 1, wherein it comprises;
a) 30 to 75 wt.%, preferably 50 to 60 wt.% of active ingredients as levodopa, carbidopa and entacapone or pharmaceutically acceptable salts or hydrates,
b) 1 to 10 wt.%, preferably 3 to 5 wt.% of wet granulation binder,
c) 15 to 40 wt.% of diluents,
d) 0 to 30 wt.%, preferably 5 to 15 wt.% of disintegrant.

4. The pharmaceutical tablet composition according to claim 1, wherein binders are selected from the group consisting of starch, gums, pregelatinized starch, polyvinyl pyrrolidone (PVP), copovidone, cellulose derivatives, such as hydroxypropylmethylcellulose, hydroxypropylcellulose, and carboxymethylcellulose and their salts.

5. The pharmaceutical tablet composition according to claim 1, wherein diluents are selected from the group consisting of lactose, lactose monohydrate, mannitol, sucrose, maltodextrin, dextrin, maltitol, sorbitol, xylitol, powdered cellulose, cellulose gum, microcrystalline cellulose, starch, calcium phosphate, or metal carbonate.

6. The pharmaceutical tablet composition according to claim 1, wherein lubricants are selected from the group consisting of talc, magnesium stearate, calcium stearate, polyethylene glycol, hydrogenated vegetable oils, stearic acid, sodium stearyl fumarate.

7. The pharmaceutical tablet composition according to claim 1, wherein disintegrants are selected from the group consisting of starch, croscarmellose sodium, crosspovidone, sodium starch glycolate and polacrilin potassium.

8. The pharmaceutical tablet composition according to any preceeding claims, which is an oral solid tablet composition in which a pharmacologically effective amount of levodopa, pharmacologically effective amounts of carbidopa or a salt, povidone, starch, crosscarmellose sodium and mannitol are premixed and granulated with water.

9. The pharmaceutical tablet composition according to any preceeding claims, which is an oral solid tablet composition in which a pharmacologically effective amount of entacapone, mannitol, starch and crosscarmellose sodium are granulated with an aqueous solution of povidone.

10. The pharmaceutical tablet composition according to any preceeding claims wherein said tablet is an immediate release film coated tablet.

## Patentansprüche

1. Pharmazeutische Tablettenzusammensetzung, umfassend Levodopa, Carbidopa und Entacapon oder pharmazeutisch verträgliche Salze oder Hydrate davon und einen pharmazeutisch verträglichen Hilfsstoff, **dadurch gekennzeichnet, dass**; Carbidopa und Levodopa zusammen granuliert sind und Entacapon separat mit einem oder mehreren pharmazeutisch verträglichen Hilfsstoffen durch das Nassgranulationsverfahren granuliert ist; wobei die mittlere Teilchengrößenverteilung d(50) von Entacapon oder pharmazeutisch verträglichen Salzen oder Hydraten etwa 2 µm Volumenäquivalent beträgt, wenn gemessen durch die Methode der Laserbeugung in einem Trockendispersionssystem.

2. Pharmazeutische Tablettenzusammensetzung nach Anspruch 1, wobei die Zusammensetzung ein Auflösungsprofil aufweist, so dass mindestens 85% Entacapon innerhalb von 15 Minuten freigesetzt werden; wobei die Freisetzungsrate in Einrichtung 1 (USP, Auflösung, Korb, 125 U/min) unter Verwendung von 900 ml Phosphatpuffer mit pH 5,5 bei 37 °C ± 0,5 °C gemessen wird.

3. Pharmazeutische Tablettenzusammensetzung, hergestellt nach Anspruch 1, umfassend;
a) 30 bis 75 Gew.%, vorzugsweise 50 bis 60 Gew.% Wirkstoffe wie Levodopa, Carbidopa und Entacapon oder pharmazeutisch verträgliche Salze oder Hydrate,
b) 1 bis 10 Gew.%, vorzugsweise 3 bis 5 Gew.% Nassgranulationsbindemittel,
c) 15 bis 40 Gew.% Verdünnungsmittel,
d) 0 bis 30 Gew.%, vorzugsweise 5 bis 15 Gew.% Zerkleinerungsmittel.

4. Pharmazeutische Tablettenzusammensetzung nach Anspruch 1, wobei Bindemittel ausgewählt sind aus der Gruppe bestehend aus Stärke, Gummis, vorgelatinierter Stärke, Polyvinylpyrrolidon (PVP), Copovidon, Zellulosederivaten wie Hydroxypropylmethylzellulose, Hydroxypropylzellulose und Carboxymethylzellulose und deren Salze.

5. Pharmazeutische Tablettenzusammensetzung nach Anspruch 1, wobei Verdünnungsmittel ausgewählt sind aus der Gruppe bestehend aus Lactose, Lactosemonohydrat, Mannit, Saccharose, Maltodextrin, Dextrin, Maltit, Sorbit, Xylit, pulverisierte Cellulose, Cellulosegummi, mikrokristallische Cellulose, Stärke, Calciumphosphat oder Metallcarbonat.

6. Pharmazeutische Tablettenzusammensetzung nach Anspruch 1, wobei Gleitmittel ausgewählt sind aus der Gruppe bestehend aus Talk, Magnesiumstearat, Calciumstearat, Polyethylenglycol, hydrierten Pflanzenölen, Stearinsäure, Natriumstearylfumarat

7. Pharmazeutische Tablettenzusammensetzung nach Anspruch 1, wobei die ZerkleinerungSmittel ausgewählt sind aus der Gruppe bestehend aus Stärke, Croscarmellose-Natrium, Crosspovidon, Natriumstärkeglykolat und Polacrilin-Kalium

8. Pharmazeutische Tablettenzusammensetzung nach einem der vorhergehenden Ansprüche, wobei sie eine orale feste Tablettenzusammensetzung ist, in der eine pharmakologisch wirksame Menge an Levodopa, pharmakologisch wirksame Mengen an Carbidopa oder einem Salz, Povidon, Stärke, Crosscarmellose-Natrium und Mannit vorgemischt und mit Wasser granuliert sind.

9. Pharmazeutische Tablettenzusammensetzung nach einem der vorhergehenden Ansprüche, wobei sie eine feste orale Tablettenzusammensetzung ist, in der eine pharmakologisch wirksame Menge von Entacapon, Mannit, Stärke und Crosscarmellose-Natrium mit einer wässrigen Lösung von Povidon granuliert wird.

10. Pharmazeutische Tablettenzusammensetzung nach einem der vorhergehenden Ansprüche, wobei die Tablette eine filmbeschichtete Tablette mit sofortiger Freisetzung ist.

## Revendications

1. Composition de comprimé pharmaceutique comprenant de la lévodopa, de la carbidopa et de l'entacapone ou leurs sels ou hydrates pharmaceutiquement acceptables et un excipient pharmaceutiquement acceptable et sa caractéristique ; la co-granulation de la carbidopa et de la lévodopa et la granulation séparée de l'entacapone avec un ou plusieurs excipients pharmaceutiquement acceptables par granulation humide, dans laquelle la distribution de la taille moyenne des particules d(50) de l'entacapone ou de ses sels ou hydrates pharmaceutiquement acceptables est lasée dans un système de dispersion sec Son équivalent volumique approximatif est de 2 µm lorsqu'il est mesuré par la méthode de diffraction.

2. Composition de comprimé pharmaceutique selon la revendication 1, dans laquelle la composition présente un profil de dissolution tel qu'au moins 85% de l'entacapone est libéré en 15 minutes ; ici, la vitesse de libération est mesurée à 37oC ± 0,5 OC dans l'appareil 1 (dissolution USP, panier, 125 rpm) en utilisant un tampon phosphate de pH 5,5 de 900 mL.

3. Composition de comprimé pharmaceutique selon la revendication 1, dans laquelle la composition comprend ;
a) Lévodopa, carbidopa et entacapone ou leurs sels ou hydrates pharmaceutiquement acceptables 30 à 75 % en poids, de préférence 50 à 60 % de principe actif,
b) 1% à 10% en poids, de préférence 3% à 5% de liant de granulation humide,
c) 15 % à 40 % de diluant en poids,
d) de 0% à 30% en poids, de préférence de 5% à 15% en poids

4. Composition de comprimé pharmaceutique selon la revendication 1, dans laquelle les liants sont choisis dans le groupe constitué par l'amidon, les gommes, l'amidon prégélatinisé, la polyvinylpyrrolidone (PVP), la copovidone, les dérivés de cellulose tels que l'hydroxypropylméthylcellulose, l'hydroxypropylcellulose et la carboxyméthylcellulose, et leurs sels.

5. Composition de comprimé pharmaceutique selon la revendication 1, dans laquelle les diluants sont choisis dans le groupe constitué par le lactose, le lactose monohydraté, le mannitol, le saccharose, la maltodextrine, la dextrine, le maltitol, le sorbitol, le xylitol, la cellulose en poudre, la gomme de cellulose, la cellulose microcristalline, l'amidon, le phosphate de calcium ou le carbonate métallique.

6. Composition de comprimé pharmaceutique selon la revendication 1, dans laquelle les lubrifiants sont choisis dans le groupe constitué par le talc, le stéarate de magnésium, le stéarate de calcium, le polyéthylène glycol, les huiles végétales hydrogénées, l'acide stéarique, le stéaryl fumarate de sodium.

7. Composition de comprimé pharmaceutique selon la revendication 1, dans laquelle les désintégrants sont choisis dans le groupe constitué par l'amidon, la croscarmellose sodique, la crospovidone, le glycolate d'amidon sodique et la polacryline potassique.

8. Composition de comprimé pharmaceutique selon l'une quelconque des revendications précédentes, dans laquelle la composition de comprimé solide oral contient une quantité pharmacologiquement efficace de lévodopa, une quantité pharmacologiquement efficace de carbidopa ou d'un sel, de la povidone, de l'amidon, de la croscarmellose sodique et du mannitol préalablement mélangés et granulés avec de l'eau.

9. Composition de comprimé pharmaceutique selon l'une quelconque des revendications précédentes, dans laquelle la composition de comprimé solide oral est granulée avec une quantité pharmacologiquement efficace d'entacapone, de mannitol, d'amidon et de croscarmellose sodique en solution aqueuse de povidone dans la composition.

10. Selon l'une quelconque des revendications précédentes, il s'agit d'une composition de comprimé pharmaceutique, et le comprimé mentionné est un comprimé pelliculé qui assure une libération immédiate.
